# EUROPEAN PATENT SPECIFICATION

(11) **EP 3 418 376 B1**
(45) Date of publication and mention of the grant of the patent: **13.03.2024**
(21) Application number: 17753324.7
(22) Date of filing: 17.02.2017
(51) Int. Cl.: A61P 1/00, C12N 5/00, C12N 5/071, C12N 5/095, C12N 5/10

(54) **CELL CULTURE MEDIUM, CULTURE METHOD, AND ORGANOID**
ZELLKULTURMEDIUM, KULTURVERFAHREN UND ORGANOID
MILIEU DE CULTURE CELLULAIRE, PROCÉDÉ DE CULTURE ET ORGANOÏDE

(30) Priority: 18.02.2016 JP 2016029060
(43) Date of publication of application: 26.12.2018
(73) Proprietor: Keio University, Tokyo 108-8345 (JP); Osaka University, Suita-shi, Osaka 565-0871 (JP)
(72) Inventor: SATO Toshiro, Tokyo 160-8582 (JP); MATANO Mami, Tokyo 160-8582 (JP); SUGIMOTO Shinya, Tokyo 160-8582 (JP); TAKAGI Junichi, Suita-shi Osaka 565-0871 (JP)
(74) Representative: HGF
(86) International application number: PCT/JP2017/005946
(87) International publication number: WO 2017/142069

(56) References cited:
- WO-A1-2015/173425
- WO-A1-2016/104646
- WO-A2-2012/168930
- WO-A2-2015/051122
- Willert ET AL: "Protocol For The Purification Of Wnt Proteins.", , 21 April 2003 (2003-04-21), XP055335829, Retrieved from the Internet: URL:http://web.stanford.edu/~rnusse/assays /W3aPurif.htm [retrieved on 2017-01-17]
- EMIKO MIHARA ET AL: "Active and water-soluble form of lipidated Wnt protein is maintained by a serum glycoprotein afamin/[alpha]-albumin", ELIFE, vol. 5, 23 February 2016 (2016-02-23), XP055477142, DOI: 10.7554/eLife.11621
- Anonymous: "Mass-production of proteins necessary for maintenance and proliferation of stem cells, the key to regenerative medicine, established - ResOU", , 19 February 2016 (2016-02-19), XP055600327, Retrieved from the Internet: URL:https://resou.osaka-u.ac.jp/en/researc h/2016/20160219_1 [retrieved on 2019-06-27]
- SATO, T. et al.: "Long-term Expansion of Epithelial Organoids From Human Colon, Adenoma, Adenocarcinoma, and Barrett's Epithelium", Gastroenterology, vol. 141, no. 5, November 2011 (2011-11), pages 1762-1772, XP028325676,
- YUKI OTA et al.: "Byori Soshiki eno Aratana Approach 1 Organoid", The Liver Cancer Journal, vol. 7, no. 2, June 2015 (2015-06), pages 24 (100)-29 (105), XP009512259, ISSN: 1883-9347
- TOSHIRO SATO: "Chokan Johi Kansaibo Baiyo Gijutsu o Donoyo ni Rinsho Oyo shite Ikuka = [How to apply intestinal epithelial stem cell culture technology to clinical use]", Molecular Gastrointestinal Medicine, vol. 9, no. 2, 2012, pages 6-10, XP009513327, ISSN: 1348-995X
- Carthy Jon M. ET AL: "Commercially Available Preparations of Recombinant Wnt3a Contain Non-Wnt Related Activities Which May Activate TGF-[beta] Signaling", Journal of Cellular Biochemistry, vol. 117, no. 4, 15 September 2015 (2015-09-15), pages 938-945, XP055884964, ISSN: 0730-2312, DOI: 10.1002/jcb.25378 Retrieved from the Internet: URL:https://onlinelibrary.wiley.com/doi/fu ll-xml/10.1002/jcb.25378>
- Anonymous: "Wnt3a, Recombinant Mouse - Certificate of Analysis Cat. #GF154", , 1 January 2014 (2014-01-01), XP055884968, Retrieved from the Internet: URL:https://www.merckmillipore.com/DE/de/p roduct/Wnt-3a-recombinant-mouse,MM_NF-GF15 4?ReferrerURL=https%3A%2F%2Fwww.google.com %2F#anchor_COA [retrieved on 2022-01-28]

## Description

### Technical Field

The present invention relates to a cell culture medium, and a culture method.

### Background Art

The intestinal tract is an organ having the largest area of contact with the external environment in the human body and has a function indispensable for maintaining life, such as digestion and absorption. Most of intestinal function is carried by the intestinal epithelium covering an inner layer thereof. The intestinal epithelium is composed of two compartments: a villus consisting of three differentiated cells (mucus producing cells, absorptive epithelial cells, and endocrine cells) and a crypt mainly consisting of undifferentiated proliferating cells. In the small intestinal crypt, Paneth cells that produce antimicrobial peptides are present at the bottom of the crypt. Recently, molecular genetic cell lineage analysis has revealed that Lgr5-positive cells (also called "crypt base columnar (CBC) cells") sandwiched between Paneth cells are intestinal epithelial stem cells. Lgr5-positive intestinal epithelial stem cells produce progenitor cells called transit amplifying cells, but these progenitor cells have no permanent self-renewal ability and are also limited to 1 to 3 lines of differentiation potential. The transit amplifying cells differentiate with 2 to 4 divisions in the crypts and terminally differentiate in the villi. These differentiated cells detach at the apex of villi and die by apoptosis. The intestinal epithelium is a rapidly metabolizing tissue and migrates from the crypt stem cells to the apex of the villi in 4 to 5 days. Unlike other differentiated cells, Paneth cells migrate to the bottom of the crypt under differentiation and have a long cell life of 2 months.

The self-renewal mechanism of intestinal epithelial stem cells is known to be controlled by Wnt signal and bone morphogenetic protein (BMP) signal from the results of several genetically modified mice. Intestinal epithelial-specific knockout mice of adenomatous polyposis coli (APC) which is a suppressor molecule of Wnt signal show intestinal epithelial cell hyperproliferation and adenomas formation. In addition, ectopic crypt formation was observed in mice overexpressing Noggin, which is an inhibitory protein of BMP, in intestinal epithelial cells, and therefore it has been suggested that the BMP signal inhibitively acts on intestinal epithelial stem cells. In fact, the Wnt signal has a high activity at the bottom of the crypt, with a gradient that is lower towards the luminal side. On the other hand, it is known that the BMP signal shows a gradient opposite to that of the Wnt signal.

In addition, long-term culture of intestinal epithelial cells has been impossible for a long time. This is considered to be due to the fact that the growth factor necessary for the maintenance of intestinal epithelial stem cells was unknown. In recent years, successful long-term maintenance of intestinal epithelial stem cells has been achieved by adhering intestinal epithelial stem cells onto an extracellular matrix and culturing the cells in the presence of a cell culture medium containing a basic medium for animal or human cells to which a BMP inhibitor, a mitogenic growth factor, and a Wnt agonist are added (see, for example, PTL 1).

WO 2015/173425 A1 (Koninklijke Nederlandse Akademie Van Wetenschappen), discloses the use of Wnt3a-conditioned medium together with R-spondin, EGF, noggin, A83-01 for growing and inducing epithelial organoids.

WO 2012/168930 A2 (Konink Akademie Van Wetenschappen Knaw), discloses epithelial organoid culture comprising Wnt3a-conditioned medium, EGF, A83-01, noggin, R-spondin-1 and SB202190 and resulting organoids and uses.

Willert et al., Protocol for the Purificaition of Wnt Proteins (21 April 2003) and WO 2015/051122 A2 (Univ Leland Stanford Junior; Dhamdhere Girija), discloses routine Wnt3a purification protocols comprising detergent.

WO 2016/104646 A1 (Osaka University) discloses Wnt-afamin complexes and serum-free medium containing purified Wnt-afamin.

Carthy Jon M et al., Commercially Available Preparations of Recombinant Wnt3a Contain Non-Wnt Related Activities Which May Activate TGF-beta signaling, Journal of Cellular Biochemistry, vol. 117, no. 4, 15 Sept 2015, pages 938-945, discloses that recombinant Wnt3a in particular cases is Wnt3a purified from conditioned medium.

### Citation List

### Patent Literature

[PTL 1] Japanese Patent No. 5458112

### Summary of Invention

The invention is defined by claims 1 and 9. Further embodiments of the invention are defined Technical Problem by the dependent claims.

Of the Wnt agonists used in the culture method described in PTL 1, the Wnt protein can be stably obtained by a supernatant obtained by culturing L cells or the like derived from mouse fibroblasts overexpressing a Wnt gene together with serum (hereinafter, referred to as "serum-containing Wnt supernatant"). Animal serum such as fetal bovine serum contained in the serum-containing Wnt supernatant is essential for the stabilization of Wnt protein. The Wnt protein purified from the serum-containing Wnt supernatant can be stabilized with a surfactant, but the purified Wnt protein has a markedly lower activity than the serum-containing Wnt supernatant. Since human cultured organoids require stimulation of highly active Wnt proteins, it is necessary to use serum-containing Wnt supernatants. However, the epithelial stem cells obtained by the conventional culture method including the serum-containing Wnt supernatant or the organoids containing the epithelial stem cells contain serum-derived impurities and the like, so it is difficult to apply them to regenerative medicine and the like, and the surfactant contained in the purified Wnt protein is cytotoxic. Further, with the conventional medium and culture method involving the Wnt supernatant, it was impossible to culture some human tissues and cancer tissues.

In order to solve the above-mentioned problems, the present invention aims to provide a cell culture medium with which serum-free long-term culture of an epithelial stem cell, an epithelial cancer cell, or a tissue containing at least one thereof can be achieved; enable the serum-free production of a human cultured organoid; and enable culture from tissues that have been impossible thus far.

### Solution to Problem

As a result of extensive research to achieve the foregoing object, the present inventors have found that a protein called afamin in serum contributes to the stabilization and solubilization of Wnt protein. The present invention has been completed based on these findings. The present inventors have focused on the fact that the Wnt protein can be stabilized in a medium in which the Wnt protein, which is a lipid-soluble protein, is water-soluble in the presence of afamin contained in serum, and have completed the present invention which is a serum-free medium containing no surfactant and a culture method using the medium, by using a medium containing Wnt protein and afamin.

That is, the present invention includes the following aspects.
[1] A serum-free cell culture medium comprising: a Wnt agonist including a complex of a Wnt protein and an afamin,
   and R-spondin; and at least one selected from the group consisting of a mitogenic growth factor, a bone morphogenetic protein (BMP) inhibitor, a transforming growth factor-β (TGF-β) inhibitor, and a p38 inhibitor.
[2] The cell culture medium according to [1], in which the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.
[3] The cell culture medium according to [1] or [2], in which the R-spondin is at least one selected from the group consisting of R-spondin 1, R-spondin 2, R-spondin 3, and R-spondin 4.
[4] The cell culture medium according to any one of [1] to [3], in which the mitogenic growth factor is an epidermal growth factor (EGF).
[5] The cell culture medium according to any one of [1] to [4], in which the BMP inhibitor is Noggin.
[6] The cell culture medium according to any one of [1] to [5], in which the TGF-β inhibitor is A83-01.
[7] The cell culture medium according to any one of [1] to [6], which is a serum-free medium.
[8] A method for culturing an epithelial stem cell, an epithelial cancer cell, or a tissue containing at least one of the cells, including: an extracellular matrix preparation step of preparing an extracellular matrix; an adhesion step of adhering the epithelial stem cell, the epithelial cancer cell, or the tissue containing at least one of the cells onto the extracellular matrix; and an organoid formation step of performing addition of the cell culture medium according to any one of [1] to [7] after the adhesion step, and culturing the epithelial stem cell, the epithelial cancer cell, or the tissue containing at least one of the cells to form an organoid.
[9] A method for culturing an epithelial stem cell or a tissue containing the epithelial stem cell, including: an extracellular matrix preparation step of preparing an extracellular matrix; an adhesion step of adhering the epithelial stem cell or the tissue containing the epithelial stem cell onto the extracellular matrix; and an organoid formation step of performing addition of the cell culture medium according to [7] after the adhesion step, and culturing the epithelial stem cell or the tissue containing the epithelial stem cell to form an organoid.

### Advantageous Effects of Invention

According to the cell culture medium of the present invention, it is possible to achieve long-term culture of an epithelial stem cell, an epithelial cancer cell, or a tissue containing at least one thereof, the cell being derived from a mammal including a human, or a tissue that could not be cultured in the past. Further, it is possible to achieve the formation of an organoid with high efficiency from at least one of the cell and the tissue.

### Brief Description of Drawings

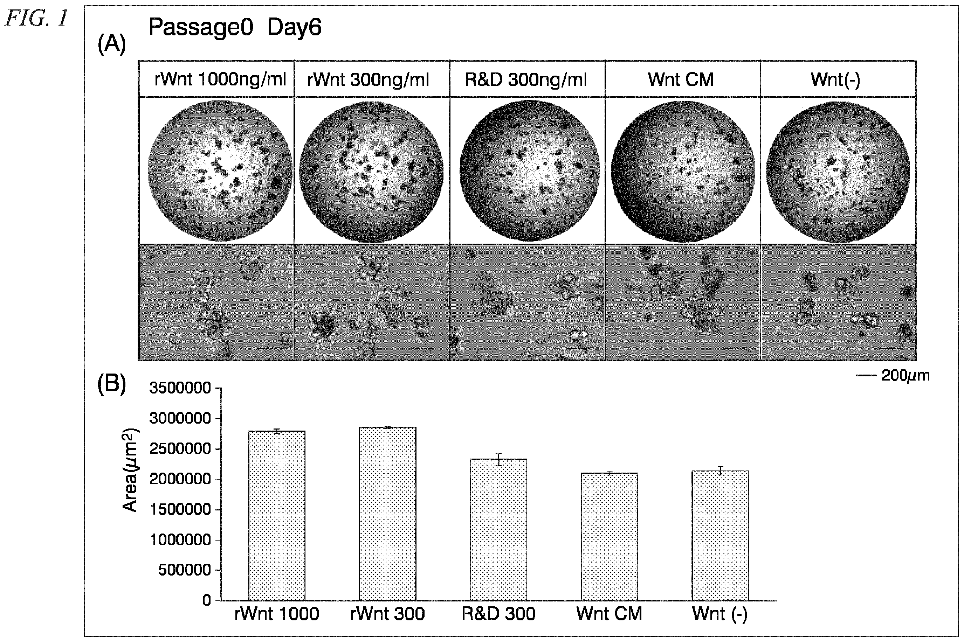

FIG. 1(A) is an image showing a state 6 days after the start of culture of a primary culture (Passage 0) of intestinal stem cells in each medium in Example 1. FIG. 1(B) is a graph obtained by quantifying the area occupied by the organoids of intestinal stem cells in wells 6 days after the start of culture of the primary culture (Passage 0) in each medium in Example 1.

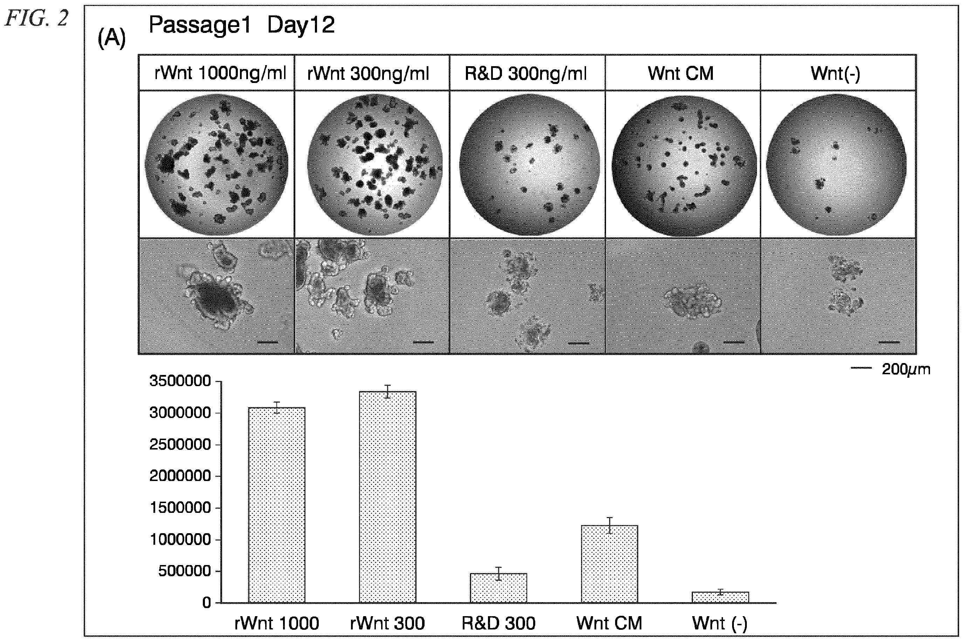

FIG. 2(A) is an image showing a state 6 days after the start of culture in a first passage (Passage 1) of intestinal stem cells in each medium in Example 1. FIG. 2(B) is a graph obtained by quantifying the area occupied by the organoids of intestinal stem cells in wells 6 days after the start of culture in the first passage (Passage 1) in each medium in Example 1.

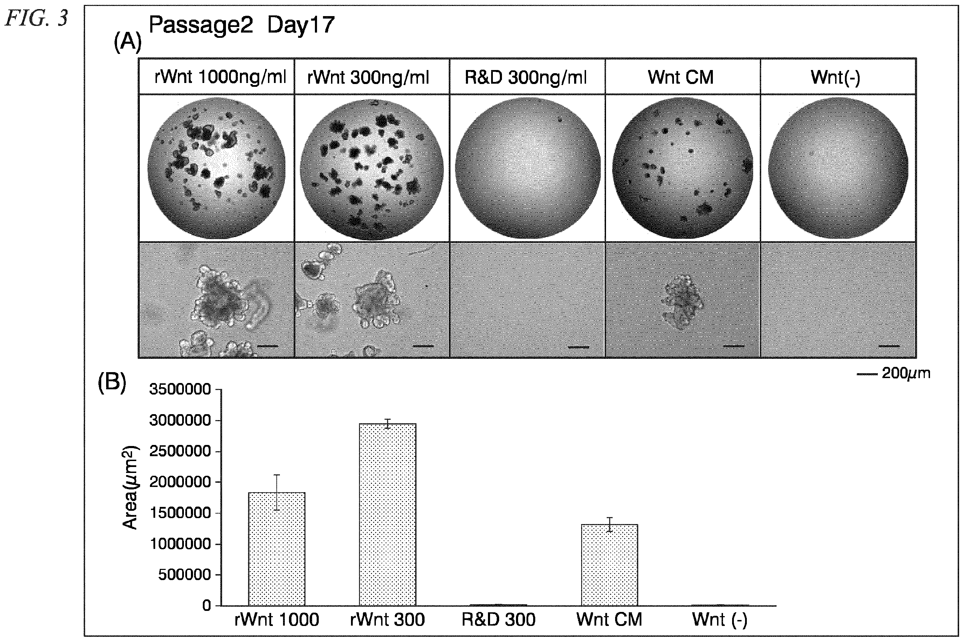

FIG. 3(A) is an image showing a state 5 days after the start of culture in a second passage (Passage 2) of intestinal stem cells in each medium in Example 1. FIG. 3(B) is a graph obtained by quantifying the area occupied by the organoids of the intestinal stem cells in wells 5 days after the start of culture in the second passage (Passage 2) in each medium in Example 1.

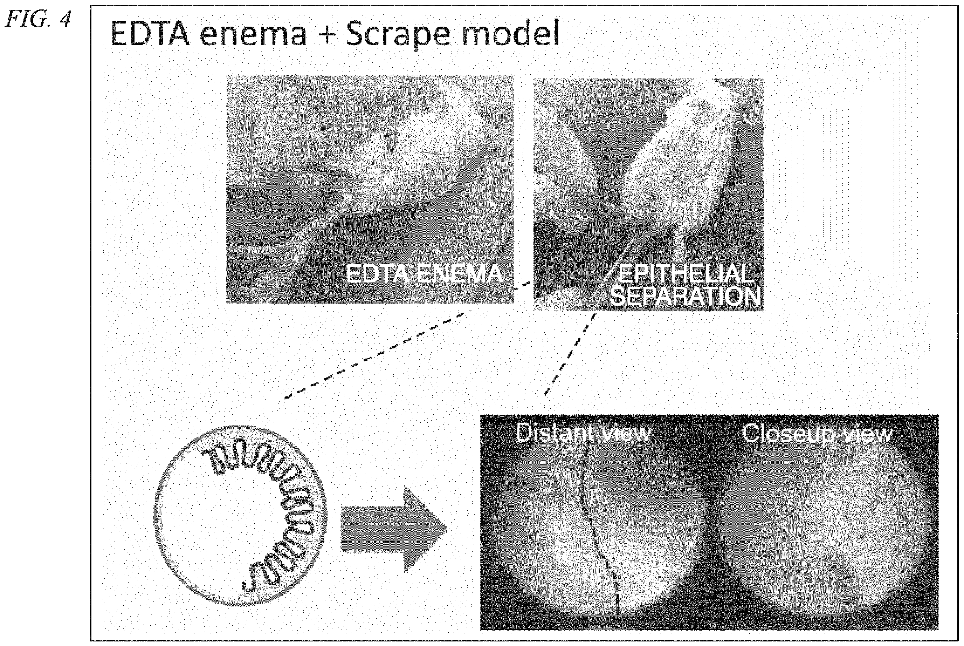

FIG. 4 is a schematic cross-sectional view and an image showing a state of colorectal mucosa exfoliation in a NOG mouse in Example 3.

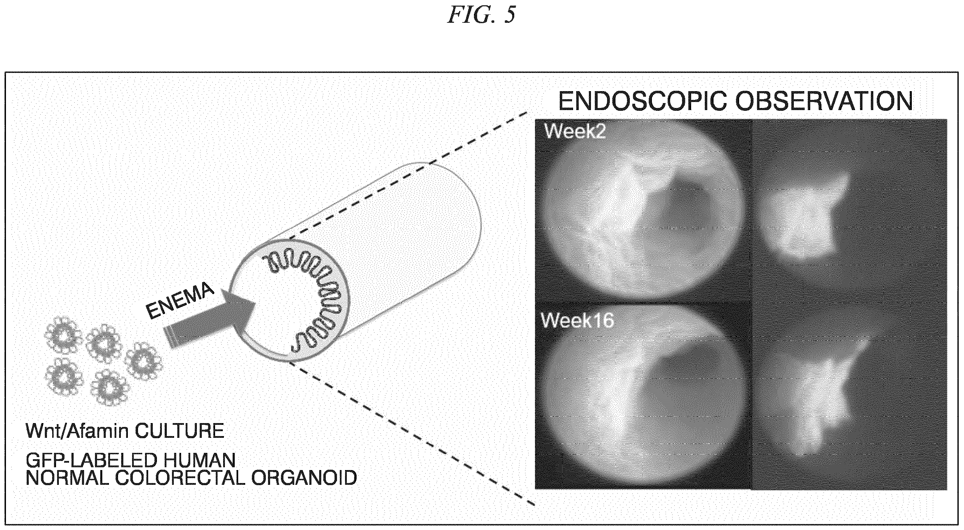

The left panel of FIG. 5 is a schematic view schematically showing the transplantation of a human colorectal organoid into the colorectal mucosa exfoliation part of the NOG mouse in Example 3. The right panel of FIG. 5 is an image in a bright field and a dark field where the transplantation site of the NOG mouse in Example 3 was observed using an endoscope at 2 weeks and 16 weeks post-transplantation.

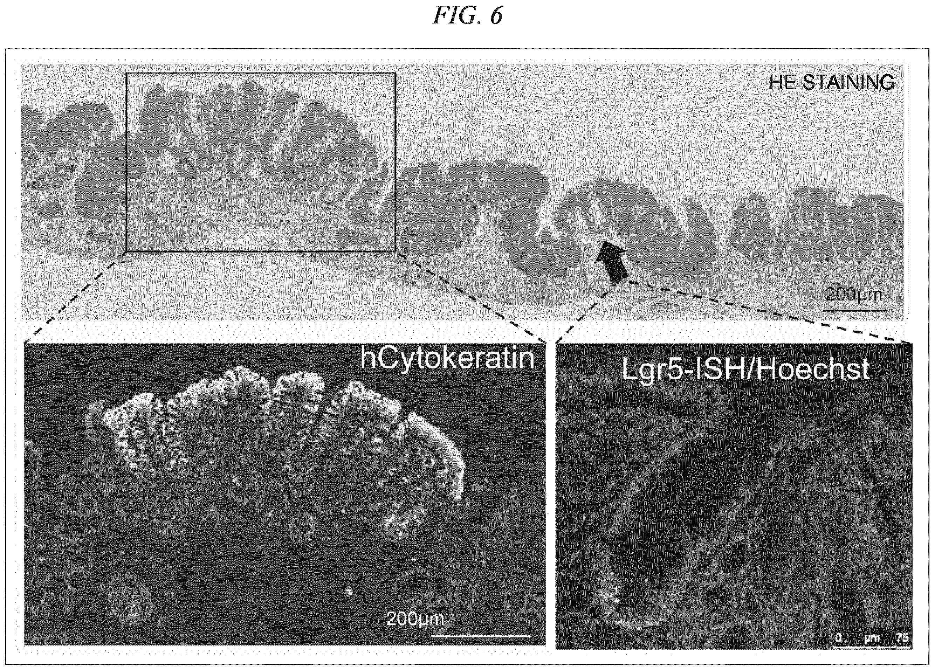

The upper panel of FIG. 6 is an image showing the results of hematoxylin-eosin (HE) staining of a tissue section of the transplantation site of the NOG mouse at 28 days post-transplantation in Example 3. The lower left panel of FIG. 6 is an image showing the results of immunostaining and nuclear staining of a tissue section of the transplantation site of the NOG mouse at 28 days post-transplantation in Example 3, with an anti-human cytokeratin antibody and Hoechst (registered trademark), respectively. The lower right panel of FIG. 6 is an image showing the results of the detection of Lgr5 by in situ hybridization analysis of a tissue section of the transplantation site of the NOG mouse at 28 days post-transplantation in Example 3, and nuclear staining of that tissue section with Hoechst (registered trademark).

### Description of Embodiments

### «Cell culture medium»

In one embodiment, the present invention provides a serum-free cell culture medium including a Wnt agonist composed of a complex of a Wnt protein and an afamin, and R-spondin, and at least one selected from the group consisting of a mitogenic growth factor, a bone morphogenetic protein (BMP) inhibitor, a transforming growth factor-β (TGF-β) inhibitor, and a p38 inhibitor.

The cell culture medium of the present embodiment is substantially free of impurities and can cultivate epithelial stem cells, epithelial cancer cells, or tissues containing at least one thereof for a long period of time. In addition, an organoid can be formed with high efficiency from epithelial stem cells, epithelial cancer cells, or tissues containing at least one thereof. In addition, epithelial stem cells cultured using the cell culture medium of the present invention can maintain a differentiation potential for a long period of time, and exhibit a very low tumor incidence.

As used herein, the term "epithelial stem cell" refers to a cell having a long-term self-renewal function and a multilineage potential into an epithelial differentiation cell, and means a stem cell derived from an epithelial tissue. Examples of the epithelial tissue include cornea, oral mucosa, skin, conjunctiva, bladder, renal tubule, kidney, digestive organs (esophagus, stomach, duodenum, small intestine (including jejunum and ileum), large intestine (including colon)), liver, pancreas, mammary gland, salivary gland, lacrimal gland, prostate, hair root, trachea, and lung. The cell culture medium of the present embodiment is particularly preferably used for culturing epithelial stem cells derived from digestive organs (esophagus, stomach, duodenum, small intestine (including jejunum and ileum), large intestine (including colon)), liver, and pancreas. As used herein, the term "epithelial cancer cell" means that the cell derived from an epithelial tissue is cancerized.

As used herein, the term "organoid" refers to a self-organized three-dimensional cellular organism by high density accumulation of cells in a controlled space.

### <Serum-free cell culture base medium>

The cell culture medium of the present embodiment includes any serum-free cell culture base medium. The cell culture medium of the present embodiment is preferably for animal cells or human cells. Such a serum-free cell culture base medium may be, for example, a defined synthetic medium buffered to pH 7.2 or more and pH 7.6 or less with a carbonate-based buffer solution. More specifically, the serum-free cell culture base medium may be, for example, a Dulbecco's Modified Eagle Medium/Ham F-12 mixed medium (Dulbecco's Modified Eagle Medium: Nutrient Mixture F-12; DMEM/F 12) supplemented with glutamine, insulin, penicillin, or streptomycin and transferrin. Also included is an RPMI 1640 medium (Roswell Park Memorial Institute 1640 medium) supplemented with glutamine, insulin, penicillin, or streptomycin and transferrin. Also included are an advanced-DMEM/F12 supplemented with glutamine and penicillin or streptomycin, and an advanced RPMI medium supplemented with glutamine and penicillin or streptomycin.

In addition, the serum-free cell culture base medium in the cell culture medium of the present embodiment may further be supplemented with a purified natural, semi-synthetic, or synthetic growth factor. Examples of the growth factor include B-27 Supplement (manufactured by Thermo Ficher Scientific Inc.), N-acetyl-L-cysteine (manufactured by Sigma-Aldrich, Inc.), and N-2 Supplement (manufactured by Thermo Ficher Scientific Inc.). These growth factors can stimulate the proliferation of some cells. The cell culture medium of the present embodiment is substantially free from an uncertain component such as fetal bovine serum (FBS) or fetal calf serum.

As used herein, the phrase "substantially free from an uncertain component" means that the cell culture medium of the present embodiment does not contain any uncertain component such as serum, that is, the cell culture medium of the present embodiment is a serum-free medium, or means a state in which the cell culture medium of the present embodiment contains only an amount of an uncertain component such as serum to the extent that it does not affect cells. In particular, the cell culture medium of the present embodiment is preferably a serum-free medium.

### <Wnt agonist>

As used herein, the term "Wnt agonist" refers to an agent which activates intracellular T-cell factor (hereinafter, also referred to as TCF)/lymphoid enhancer factor (hereinafter, also referred to as LEF)-mediated transcription. Therefore, the Wnt agonist is not limited to a Wnt family protein but includes a Wnt agonist that binds to and activates Frizzled receptor family members, an inhibitor of intracellular β-catenin degradation, and an activator of TCF/LEF. The Wnt agonist stimulates a Wnt activity in the cell such that it has at least 1.1-fold, preferably at least 1.2-fold, more preferably at least 10-fold, still more preferably at least 50-fold, particularly preferably at least 100-fold, and most preferably at least 300-fold Wnt activity level as compared to the level of Wnt activity in the absence of the Wnt agonist.

The Wnt activity can be examined by measuring the transcriptional activity of Wnt using methods known to those skilled in the art, for example, by pTOPFLASH and pFOPFLASH Tcf luciferase reporter constructs (reference: Korinek et al., 1997. Science 275: 1784-1787).

In culturing an epithelial stem cell, an epithelial cancer cell, or a tissue containing at least one thereof, it is essential that a Wnt agonist is included. The Wnt agonist contained in the cell culture medium of the present embodiment is preferably at least one of a complex of Wnt protein and afamin and R-spondin, and it is more preferred that both a complex of Wnt protein and afamin and R-spondin are included. In the cell culture medium of the present embodiment, by including the complex of Wnt protein and afamin and R-spondin, epithelial stem cells can form organoids with higher efficiency.

### [Wnt protein]

With regard to the Wnt protein which is one type of Wnt agonist, the origin thereof is not particularly limited, and Wnt proteins derived from various organisms can be used. Among them, a Wnt protein derived from a mammal is preferable. Examples of the mammal include a human, a mouse, a rat, a cow, a pig, and a rabbit. Examples of the mammalian Wnt protein include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. In the cell culture medium of the present embodiment, a plurality of Wnt proteins may be used in combination.

The method for producing a Wnt protein may be, for example, a method of producing a Wnt protein using a Wnt protein-expressing cell. With regard to the Wnt protein-expressing cell, the origin (biological species, culture form, or the like) of the cell is not particularly limited. The Wnt protein-expressing cell may be any cell that stably expresses the Wnt protein and may also be a cell that transiently expresses the Wnt protein. Examples of the Wnt protein-expressing cell include an L cell that stably expresses mouse Wnt3a (ATCC CRL-2647), and an L cell that stably expresses mouse Wnt5a (ATCC CRL-2814). In addition, the Wnt protein-expressing cell can be produced using known genetic recombination techniques. That is, the Wnt protein-expressing cell can be produced by inserting DNA encoding a desired Wnt protein into a known expression vector, and introducing the resulting expression vector into an appropriate host cell. The base sequence of a gene encoding a desired Wnt protein can be obtained from a known database such as GenBank.

The Wnt protein expressed by the Wnt protein-expressing cell may be a Wnt protein fragment or may contain an amino acid sequence other than the amino acid sequence of the Wnt protein as long as it has the Wnt activity. The amino acid sequence other than the amino acid sequence of the Wnt protein is not particularly limited, and examples thereof include an amino acid sequence of an affinity tag. The amino acid sequence of the Wnt protein need not be completely identical with the amino acid sequence that can be obtained from a known database such as GenBank. The amino acid sequence of the Wnt protein may be an amino acid sequence essentially identical to that can be obtained from a known database as long as it has the Wnt activity.

The amino acid sequence essentially identical to that of a Wnt protein that can be obtained from a known database such as GenBank may be, for example, an amino acid sequence that is identical to that of a Wnt protein obtainable from a known database except for having deletion, substitution or addition of one to several amino acids. As used herein, the phrase "having deletion, substitution or addition of one to several amino acids" means having deletion, substitution or addition of amino acid(s) of which the number corresponds to the number of amino acids that can be deleted, substituted or added by a known method for producing a mutant peptide, such as site-directed mutagenesis (preferably 10 or less amino acids, more preferably 7 or less amino acids, and still more preferably 6 or less amino acids). In addition, the amino acid sequence essentially identical to that of Wnt protein obtainable from a known database may be, for example, an amino acid sequence having identity of at least 80% or more, preferably at least 85% or more, more preferably at least 90% or more, still more preferably at least 92% or more, particularly preferably at least 95% or more, and most preferably at least 99% or more with that of Wnt protein obtainable from a known database.

The activity of the Wnt protein can be confirmed by, for example, TCF reporter assay. The TCF reporter assay is a simple method for the evaluation of the strength of Wnt protein activity. Specifically, a luciferase gene fused to a sequence for binding of T-cell factor (TCF), which is a transcription factor specifically activated upon the initiation of Wnt signal transduction in cells, is introduced, and the luminescence in the luciferase-mediated reaction is measured as an index of the strength of Wnt protein activity (Molenaar et al., Cell, 86, 391 (1996)). Evaluation methods other than the TCF reporter assay include a method using western blotting for the quantitative determination of intracellular β-catenin, which is stabilized upon the initiation of Wnt signal transduction in cells (reference: Shibamoto et al., Gene to cells, 3, 659 (1998)). Moreover, in the case where the Wnt protein is a Wnt protein that transduces a signal to cells through what is called the non-canonical pathway, for example, Wnt5a, the Wnt protein activity can be evaluated by, for example, a method involving monitoring the phosphorylation of the intracellular adaptor protein Dv12 (reference: Kikuchi et al., EMBO J. 29, 3470 (2010)).

### [R-spondin]

As the R-spondin which is one type of Wnt agonist, an R-spondin family consisting of R-spondin 1, R-spondin 2, R-spondin 3, and R-spondin 4 can be mentioned. The R-spondin family is a secretory protein and is known to be involved in the activation and regulation of the Wnt signaling pathway. In the cell culture medium of the present embodiment, a plurality of R-spondins may be used in combination.

The R-spondin may be a fragment of R-spondin or may contain an amino acid sequence other than the amino acid sequence of R-spondin, as long as it has the R-spondin activity.

### [Content]

The concentration of the Wnt protein contained in the cell culture medium of the present embodiment is 50 ng/mL or more, preferably 100 ng/mL or more and 10 µg/mL or less, more preferably 200 ng/mL or more and 1 µg/mL or less, and still more preferably 300 ng/mL or more and 1 µg/mL or less. During the culture of epithelial stem cells, preferably the Wnt agonist is added to the culture medium every 2 days, and preferably the cell culture medium is changed with a fresh culture medium every 4 days.

### <Afamin>

As used herein, the term "afamin" refers to a glycoprotein belonging to an albumin family and is known to exist in blood or body fluids. The serum usually added to the medium used for cell culture contains afamin derived from an animal from which the serum has been collected. In the cell culture medium of the present embodiment, it is preferable to use afamin alone without using serum, since the serum contains impurities and the like other than afamin.

The origin of afamin contained in the cell culture medium of the present embodiment is not particularly limited, and afamin derived from various organisms can be used. Among them, a mammal-derived afamin is preferable. Examples of the mammal are the same as those in the foregoing section [Wnt protein]. The amino acid sequences of the major mammalian afamins and the base sequences of the genes encoding those mammalian afamins can be obtained from a known database such as GenBank. For example, in GenBank, the amino acid sequence of human afamin is registered under the accession number AAA21612, and the base sequence of the gene encoding the human afamin is registered under the accession number L32140; and the amino acid sequence of bovine afamin is registered under the accession number DAA28569, and the base sequence of the gene encoding the bovine afamin is registered under the accession number GJ060968.

The afamin contained in the cell culture medium of the present embodiment may be a product obtained by purifying natural afamin contained in serum or the like by a known method or recombinant afamin. The recombinant afamin can be produced by appropriately using a known gene recombination technique. For example, the recombinant afamin can be produced by a method in which DNA encoding afamin is inserted into a known expression vector, the resulting expression vector is introduced into an appropriate host cell to express recombinant afamin, and the expressed recombinant afamin is purified using a known purification method. The recombinant afamin may be an afamin with an affinity tag added thereto. The affinity tag to be added is not particularly limited, and it can be appropriately selected from known affinity tags and then used. The affinity tag is preferably an affinity tag which is recognized by a specific antibody, and examples thereof include FLAG tab, MYC tag, HA tag, and V5 tag.

The above-mentioned Wnt protein is strongly hydrophobic due to fatty acid (palmitoleic acid) modification of a specific serine residue, so that the protein tends to aggregate and denature in an aqueous solution. For this reason, Wnt proteins are widely known to be quite difficult to purify and store. On the other hand, this fatty acid modification of a specific serine residue is reportedly essential for the physiological activity of Wnt proteins and involved in binding of Wnt proteins to Frizzled receptor family members. The present inventors have found that Wnt protein binds to afamin in a 1:1 ratio to form a complex in an aqueous solution and solubilizes while maintaining high physiological activity.

Based on such findings, a Wnt protein-afamin complex may be produced by a method of culturing a cell expressing both the Wnt protein and afamin or the Wnt protein-afamin complex may be produced by a method of co-culturing a Wnt protein-expressing cell and an afamin-expressing cell. The activity of the Wnt protein in the Wnt protein-afamin complex can be evaluated using the same method as described in the foregoing section [Wnt protein].

The concentration of afamin contained in the cell culture medium of the present embodiment is not particularly limited, but it is preferably 50 ng/mL or more and 10 µg/mL or less, more preferably 100 ng/mL or more and 1 µg/mL or less, and still more preferably 300 µg/mL or more and 1 µg/mL or less.

### <Mitogenic growth factor>

Examples of the mitogenic growth factor contained in the cell culture medium of the present embodiment include a family of growth factors such as an epidermal growth factor (EGF), a transforming growth factor-α (TGF-α), a basic fibroblast growth factor (bFGF), a brain derived neurotrophic factor (BDNF), and a keratinocyte growth factor (KGF). A plurality of the mitogenic growth factors may be used in combination.

The EGF is a potent mitogen for various cultured ectodermal and mesodermal cells and has a significant effect on the differentiation of some fibroblasts and of specific cells. The EGF precursor exists as a membrane-bound molecule that is proteolytically cleaved to produce a 53-amino acid peptide hormone that stimulates cells.

Among the mitogenic growth factors contained in the cell culture medium of the present embodiment, EGF is particularly preferable. The concentration of EGF contained in the cell culture medium of the present embodiment is preferably 5 ng/mL or more and 500 ng/mL or less, more preferably 10 ng/mL or more and 400 ng/mL or less, and still more preferably 50 ng/mL or more and 200 ng/mL or less.

Also in the case where bFGF (preferably FGF10 or FGF7) is contained in the cell culture medium of the present embodiment, it is preferred that the content of bFGF is the same as that of EGF. In the case where a plurality of FGFs such as FGF7 and FGF10 are used, it is preferred that the total content of FGFs is within the above range. During the culture of the stem cells, it is preferable to add the mitogenic growth factor to the culture medium every 2 days, and it is preferable to change the culture medium with a fresh culture medium every 4 days.

### <BMP inhibitor>

The bone morphogenetic protein (BMP) is a dimeric protein and binds to a receptor complex consisting of two types of serine/threonine kinase receptors, a type I receptor and a type II receptor. The type II receptor phosphorylates the type I receptor, resulting in the activation of this receptor kinase. This type I receptor subsequently phosphorylates a specific receptor substrate (SMAD), resulting in the transcriptional activity being guided by the signal transduction pathway. Generally, the BMP inhibitor is, for example, an agent that blocks or inhibits the binding of a BMP molecule to a BMP receptor and binds to a BMP molecule to form a complex which neutralizes the BMP activity. In addition, the BMP inhibitor is, for example, an agent that binds to a BMP receptor and blocks or inhibits the binding of a BMP molecule to a receptor and acts as an antagonist or inverse agonist.

The BMP inhibitor has an inhibitory activity of preferably 50% or more, more preferably 70% or more, still more preferably 80% or more, and particularly preferably 90% or more, as compared to the level of BMP activity in the absence of the inhibitor. The BMP inhibitory activity can be evaluated by measuring the transcriptional activity of BMP using a method known to those skilled in the art (reference: Zilberberg et al., BMC Cell Biol, 8: 41, 2007).

The BMP inhibitor contained in the cell culture medium of the present embodiment is preferably a naturally occurring BMP-binding protein, examples of which include a Chordin-like protein such as Noggin, Chordin, or a Chordin domain; a Follistatin-related protein such as Follistatin or a Follistatin domain; a DAN-like protein such as DAN or a DAN cysteine-knot domain; Sclerostin/SOST, Decorin, and α-2 macroglobulin. As the BMP inhibitor contained in the cell culture medium of the present embodiment, among them, a Chordin-like protein or a DAN-like protein is preferable, and a Chordin-like protein is more preferable. Noggin is preferred as the Chordin-like protein. The Chordinin-like protein and the DAN-like protein are diffusible proteins and can bind to BMP molecules with varying degrees of affinity to inhibit access of BMP molecules to signaling receptors. In the case of culturing epithelial stem cells, the addition of these BMP inhibitors to the cell culture medium can prevent the loss of stem cells.

The concentration of the BMP inhibitor contained in the cell culture medium of the present embodiment is preferably 10 ng/mL or more and 100 ng/mL or less, more preferably 20 ng/mL or more and 100 ng/mL or less, and still more preferably 50 ng/mL or more and 100 ng/mL or less. During the culture of the stem cells, it is preferable to add a mitogenic growth factor to the culture medium every 2 days, and it is preferable to change the culture medium with a fresh culture medium every 4 days.

### <TGF-β inhibitor>

The transforming growth factor-β (TGF-β) is a type of growth factor and is produced in almost all cells such as kidney, bone marrow, and platelet. There are five subtypes (β1 to β5) in TGF-β. It is also known that TGF-β promotes the proliferation of osteoblasts and the synthesis and proliferation of connective tissues such as collagen and exhibits an inhibitory effect against epithelial cell proliferation and osteoclasts. In general, a TGF-β inhibitor is, for example, an agent that blocks or inhibits the binding of TGF-β to a TGF-β receptor and binds to TGF-β to form a complex which neutralizes the TGF-β activity. In addition, the TGF-β inhibitor is, for example, an agent that binds to the TGF-β receptor to block or inhibit the binding of TGF-β to the receptor and acts as an antagonist or inverse agonist.

The TGF-β inhibitor has an inhibitory activity of preferably 50% or more, more preferably 70% or more, still more preferably 80% or more, and particularly preferably 90% or more, as compared to the level of TGF-β activity in the absence of this inhibitor. The TGF-β inhibitory activity can be evaluated by a method known to those skilled in the art. Such an evaluation system may be, for example, a cellular assay in which cells are stably transfected with a reporter construct containing the human PAI-1 promoter or Smad-binding site, driving a luciferase reporter gene (reference: De Gouville et al., Br J Pharmacol, 145 (2): 166-177, 2005.).

### Examples of the TGF-β inhibitor contained in the cell culture medium of the present embodiment include A83-01

(3-(6-methylpyridin-2-yl)-1-phenylthiocarbamoyl-4-quinolin-4-ylpyrazole), ALK5 Inhibitor I (3-(pyridin-2-yl)-4-(4-quinonyl)-1H-pyrazole), LDN 193189 (4-(6-(4-(piperazin-1-yl)phenyl)pyrazolo[1,5-a]pyrimidin-3-yl)quinoline), SB 431542 (4-[4-(1,3-benzodioxol-5-yl)-5-pyridin-2-yl]-1H-imidazol-2-yl]benzamide), SB-505124 (2-(5-benzo[1,3]dioxol-5-yl-2-tert-butyl-3H-imidazol-4-yl)-6-methylpyridine hydrochloride hydrate), SD-208 (2-(5-chloro-2-fluorophenyl)pteridin-4-yl)pyridin-4-yl-amine), SB-525334 (6-[2-(1,1-dimethylethyl)-5-(6-methyl-2-pyridinyl)-1H-imidazol-4-yl]quinoxaline), LY-364947 (4-[3-(2-pyridinyl)-1H-pyrazol-4-yl]-quinoline), LY 2157299 (4-[2-(6-methyl-pyridin-2-yl)-5,6-dihydro-4H-pyrrolo[1,2-b]pyrazol-3-yl]-quinoline-6-ca rboxylic acid amide) TGF-β RI Kinase Inhibitor II 616452 (2-(3-(6-methylpyridin-2-yl)-1H- pyrazol-4-yl)-1,5-naphthyridine), TGF-β RI Kinase Inhibitor III 616453 (2-(5-benzo[1,3]dioxol-4-yl-2-tert-butyl-1H-imidazol-4-yl)-6-methylpyridine, HCl), TGF-β RI Kinase Inhibitor IX 616463 (4-((4-((2,6-dimethylpyridin-3-yl)oxy)pyridin-2-yl)amino)benzenesulfonamide), TGF-β RI Kinase Inhibitor VII 616458 (1-(2-((6,7-dimethoxy-4-quinolyl)oxy)-(4,5-dimethylphenyl)-1-ethanone), TGF-β RI Kinase Inhibitor VIII 616459 (6-(2-tert-butyl-5-(6-methyl-pyridin-2-yl)-1H-imidazol-4-yl)-quinoxaline), AP 12009 (TGF-β2 antisense compound "Trabedersen"), Belagenpumatucel-L (TGF-β2 antisense gene-modified allogeneic tumor cell vaccine), CAT-152 (Glaucoma-lerdelimumab (Anti-TGF-β-2 monoclonal antibody)), CAT-192 (Metelimumab (human IgG4 monoclonal antibody that neutralizes TGFβ1), and GC-1008 (anti-TGF-β monoclonal antibody). As the TGF-β inhibitor contained in the cell culture medium of the present embodiment, A83-01 is preferable among them.

The concentration of the TGF-β inhibitor contained in the cell culture medium of the present embodiment is preferably 100 nM or more and 10 µM or less, more preferably 500 nM or more and 5 µM or less, and still more preferably 500 nM or more and 2 µM or less. During the culture of the stem cells, it is preferable to add the TGF-β inhibitor to the culture medium every 2 days, and it is preferable to change the culture medium with a fresh culture medium every 4 days.

### <p38 inhibitor>

As used herein, the term "p38 inhibitor" refers to any inhibitor that directly or indirectly negatively regulates p38 signaling. Generally, the p38 inhibitor binds to p38, for example, and reduces the activity thereof. The p38 protein kinase is part of the mitogen-activated protein kinase (MAPK) family. The MAPK is a serine/threonine-specific protein kinase that responds to extracellular stimuli such as environmental stress and inflammatory cytokines and regulates various cellular activities such as gene expression, mitosis, differentiation, proliferation, and cell survival/apoptosis. The p38 MAPK exists as α, β, β2, γ, and δ isoforms. In addition, the p38 inhibitor is also an agent that binds to, for example, at least one p38 isoform and reduces the activity thereof.

The p38 inhibitor has an inhibitory activity of preferably 50% or more, more preferably 70% or more, still more preferably 80% or more, and particularly preferably 90% or more, as compared to the level of p38 activity in the absence of this inhibitor. The inhibitory effect by the p38 inhibitor can be evaluated by a method known to those skilled in the art. Examples of such an evaluation system include a phosphorylation site-specific antibody detection method of Thr 180/Tyr 182 phosphorylation, a biochemical recombinant kinase assay, a tumor necrosis factor α (TNF-α) secretion assay, a DiscoverRx high throughput screening platform for p38 inhibitor, and a p38 activity assay kit (for example, manufactured by EMD Millipore Corporation, Sigma-Aldrich, Inc., or the like).

### Examples of the p38 inhibitor contained in the cell culture medium of the present embodiment include SB-202190

(4-(4-fluorophenyl)-2-(4-hydroxyphenyl)-5-(4-pyridyl)-1H-imidazole), SB-203580 (4-[4-(4-fluorophenyl)-2-[4-(methylsulfinyl)phenyl]-1H-imidazol-5-yl]pyridine), VX-702 (6-(N-carbamoyl-2,6-difluoroanilino)-2-(2,4-difluorophenyl)pyridine-3-carboxamide), VX-745 (5-(2,6-dichlorophenyl)-2-[2,4-difluorophenyl)thio]-6H-pyrimido[1,6-b]pyridazin-6-one), PD-169316 (4-(4-fluorophenyl)-2-(4-nitrophenyl)-5-(4-pyridyl)-1H-imidazole), RO-4402257 (6-(2,4-difluorophenoxy)-2-{[3-hydroxy-1-(2-hydroxyethyl)propyl]amino}-8-methylpyri do[2,3-D]pyrimidin-7(8h)-one), and BIRB-796 (1-[5-tert-butyl-2-(4-methylphenyl)pyrazol-3-yl]-3-[4-(2-morpholin-4-ylethoxy)naphthal en-1-yl]urea).

The concentration of the p38 inhibitor contained in the cell culture medium of the present embodiment is preferably 50 nM or more and 100 µM or less, more preferably 100 nM or more and 50 µM or less, and still more preferably 100 nM or more and 10 µM or less. During the culture of the stem cells, it is preferable to add the p38 inhibitor to the culture medium every 2 days, and it is preferable to change the culture medium with a fresh culture medium every 4 days.

### <Other ingredients>

The cell culture medium of the present embodiment may further contain a Rock (Rho-kinase) inhibitor. Examples of the Rock inhibitor include Y-27632 ((R)-(+)-trans-4-(1-aminoethyl)-N-(4-pyridyl)cyclohexanecarboxamide dihydrochloride monohydrate), Fasudil (HA1077) (5-(1,4-diazepan-1-ylsulfonyl)isoquinoline), and H-1152 ((S)-(+)-2-methyl-1-[(4-methyl-5-isoquinolmyl)sulfonyl]-hexahydro-1H-1,4-diazepine dihydrochloride). In the case where Y-27632 is used as the Rock inhibitor, it is preferable to add Y-27632 in the first two days of culturing stem cells dispersed in a single cell. It is preferred that the concentration of Y-27632 contained in the cell culture medium of the present embodiment is 10 µM.

The cell culture medium of the present embodiment may further contain a Notch agonist. Notch signaling is known to play an important role in cell survival and proliferation. Examples of the Notch receptor protein include receptor proteins capable of interacting with numerous surface-bound or secretory ligands including Delta 1, Jagged 1 and 2, and Delta-like 1, Delta-like 3, and Delta-like 4. As the ligand binds to the receptor, the Notch receptor is activated by serial cleavage reactions by proteases involving members of the ADAM protease family and intramembrane cleavage regulated by γ-secretase/presenilin. As a result, the intracellular domain of Notch translocates to the nucleus, where it activates the transcription of downstream genes. The Notch agonist is preferably Jagged 1, Delta 1, or an active fragment or derivative thereof. The Notch agonist is more preferably a DSL peptide (reference: Dontu et al., Breast Cancer Res, 6: R605-R615, 2004.).

In the case where the Notch agonist contained in the cell culture medium of the present embodiment is a DSL peptide, the concentration thereof is preferably 10 ng/mL or more and 100 ng/mL or less. In particular, in the case where the Notch agonist is added during the first week of culture, the culture efficiency increases by 2 to 3 times. This Notch agonist is preferably added to the culture medium every 2 days for the first 7 days of the culture of this stem cell.

The Notch agonist is a molecule which stimulates the Notch activity in cells, by at least 10%, more preferably at least 20%, still more preferably at least 30%, even more preferably at least 50%, particularly preferably at least 90%, and most preferably 100%, as compared to the level of Notch activity in the absence of this agonist molecule. The Notch activity can be evaluated by measuring the transcriptional activity of Notch using a 4xwtCBF1-luciferase reporter construct by a method known to those skilled in the art (reference: Hsieh et al., Mol Cell Biol., 16, 952-959, 1996).

The cell culture medium of the present embodiment may further contain a γ-secretase inhibitor such as DAPT or DBZ. The γ-secretase inhibitor can influence cell fate determination during differentiation of cells. The concentration of the γ-secretase inhibitor contained in the cell culture medium of the present embodiment may be, for example, 1 ng/mL or more and 10 µg/mL, for example, 1 ng/mL or more and 1 µg/mL or less, or for example, 1 ng/mL or more and 100 ng/mL or less.

The cell culture medium of the present embodiment is further supplemented with gastrin (or a suitable alternative such as Leu 15-gastrin). The concentration of gastrin (or a suitable substitute) contained in the cell culture medium of the present embodiment may be, for example, 1 ng/mL or more and 10 µg/mL, for example, 1 ng/mL or more and 1 µg/mL or less, or for example, 5 ng/mL or more and 100 ng/mL or less.

The cell culture medium of the present embodiment may further contain nicotinamide. Inclusion of nicotinamide can improve, for example, culturing efficiency and lifespan of human colonic organoids. The concentration of nicotinamide contained in the cell culture medium of the present embodiment may be, for example, 1 mg/mL or more and 100 mg/mL, for example, 5 mg/mL or more and 50 mg/mL or less, or for example, 5 mg/mL or more and 20 mg/mL or less.

The cell culture medium of the present embodiment may further contain at least one amino acid. Examples of the amino acid contained in the cell culture medium of the present embodiment include L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine, and combinations thereof. Generally, the concentration of L-glutamine contained in the cell culture medium is 0.05 g/L or more and 1 g/L or less (usually 0.1 g/L or more and 0.75 g/L or less). The concentration of other amino acids contained in the cell culture medium is 0.001 g/L or more and 1 g/L (usually 0.01 g/L or more and 0.15 g/L or less). The amino acid may be of synthetic origin.

The cell culture medium of the present embodiment may further contain at least one vitamin. Examples of the vitamin contained in the cell culture medium of the present embodiment include thiamine (vitamin B1), riboflavin (vitamin B2), niacin (vitamin B3), calcium D-pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α tocopherol (vitamin E), biotin (vitamin H), and menadione (vitamin K).

The cell culture medium of the present embodiment may further contain at least one inorganic salt. The inorganic salt contained in the cell culture medium of the present embodiment is to aid maintenance of the osmotic balance of the cells and to help regulate the membrane potential. Specific examples of the inorganic salt include salts of calcium, copper, iron, magnesium, potassium, sodium, and zinc. The salts are usually used in the form of chlorides, phosphates, sulfates, nitrates, and bicarbonates. More specific examples of the salts include CaCl₂, CuSO₄-5H₂O, Fe(NO₃)-9H₂O, FeSO₄-7H₂O, MgCl, MgSO₄, KCl, NaHCO₃, NaCl, Na₂HPO₄, Na₂HPO₄-H₂O, and ZnSO₄-7H₂O.

The cell culture medium of the present embodiment may further contain a sugar which can serve as at least one carbon energy source. Examples of the sugar contained in the cell culture medium of the present embodiment include glucose, galactose, maltose, and fructose. Among them, the sugar is preferably glucose and particularly preferably D-glucose (dextrose). The concentration of the sugar contained in the cell culture medium of the present embodiment is preferably 1 g/L or more and 10 g/L.

The cell culture medium of the present embodiment may further contain at least one trace element. Examples of the trace element contained in the cell culture medium of the present embodiment include barium, bromium, cobalt, iodine, manganese, chromium, copper, nickel, selenium, vanadium, titanium, germanium, molybdenum, silicon, iron, fluorine, silver, rubidium, tin, zirconium, cadmium, zinc, aluminum, and ions thereof.

The cell culture medium of the present embodiment may further contain at least one additional agent. Examples of such an agent include nutrients or growth factors that have been reported to improve stem cell culture, such as cholesterol/transferrin/albumin/insulin/progesterone, putrescine, and selenite/other factors.

### <<Method for culturing epithelial stem cell, epithelial cancer cell, or tissue containing these cells>>

In one embodiment, the present invention provides a method for culturing an epithelial stem cell, an epithelial cancer cell, or a tissue containing at least one thereof, including: an extracellular matrix preparation step of preparing an extracellular matrix; an adhesion step of adhering the epithelial stem cell, the epithelial cancer cell, or the tissue containing such a cell onto the extracellular matrix; and an organoid formation step of performing addition of the foregoing cell culture medium after the adhesion step, and culturing the epithelial stem cell, the epithelial cancer cell, or the tissue containing such a cell to form an organoid.

According to the culture method of the present embodiment, it is possible to achieve long-term culture of an epithelial stem cell, an epithelial cancer cell, or a tissue containing at least one thereof. Further, it is possible to achieve the formation of an organoid with high efficiency from an epithelial stem cell, an epithelial cancer cell, or a tissue containing at least one thereof.

The method of the present embodiment will be described in detail below.

### [Extracellular matrix preparation step]

Generally, the term "extracellular matrix (ECM)" refers to a supramolecular structure existing outside a cell in an organism. The ECM becomes a scaffold for an epithelial stem cell, an epithelial cancer cell, or a tissue containing them to proliferate.

The ECM contains a variety of polysaccharides, water, elastin, and glycoprotein. Examples of the glycoprotein include collagen, entactin (nidogen), fibronectin, and laminin.

As a preparation method of ECM, for example, a method using connective tissue cells can be mentioned. More specifically, ECM can be used as a scaffold by culturing ECM-producing cells, for example, fibroblasts, then taking out these cells and adding epithelial stem cells, epithelial cancer cells, or a tissue containing these cells.

Examples of ECM-producing cells include chondrocytes producing mainly collagen and proteoglycan; fibroblasts producing mainly type IV collagen, laminin, interstitial procollagen, and fibronectin; and colonic myofibroblasts producing mainly collagen (type I, III, and V), chondroitin sulfate proteoglycan, hyaluronic acid, fibronectin, and tenascin-C. Alternatively, commercially available ECMs may be used. Examples of commercially available ECMs include an extracellular matrix protein (manufactured by Invitrogen Corporation) and a basement membrane preparation derived from Engelbreth-Holm-Swarm (EHS) mouse sarcoma cells (for example, Matrigel^{™} (manufactured by BD Biosciences, Inc.)). A synthetic ECM such as ProNectin (Sigma Z378666) may be used. Also, a mixture of natural ECM and synthetic ECM may be used.

The use of an ECM for culturing stem cells can enhance long-term survival of the stem cells and the continued presence of undifferentiated stem cells. In the absence of an ECM, stem cell cultures cannot be cultured over a long period of time and no continued presence of undifferentiated stem cells is observed. In addition, the presence of an ECM allows the culturing of organoids which cannot be cultured in the absence of an ECM.

The ECM is usually sunk on the bottom of the dish in which cells are suspended. For example, in the case where the ECM solidifies at 37°C, the cell culture medium described above may be added and diffused into the ECM which is then used. The cells in the medium are capable of sticking to the ECM by interaction with its surface structure, for example interaction with integrins.

The ECM may be coated on a culture vessel or the like which is then used. In the case of using fibronectin as the ECM, the culture vessel is coated with fibronectin in an amount of preferably 1 µg/cm² or more and 250 µg/cm² or less, more preferably 1 µg/cm² or more and 150 µg/cm² or less, and still more preferably 8 µg/cm² or more and 125 µg/cm² or less.

### [Adhesion step]

Subsequently, an epithelial stem cell, an epithelial cancer cell, or a tissue containing at least one thereof is prepared. The epithelial stem cells or epithelial cancer cells used in the culture method of the present embodiment may be the same as those described in the foregoing section «Cell culture medium».

Methods for isolating cells from an epithelial tissue include methods known in the art. For example, crypts can be isolated by incubation of the isolated tissue with a chelating agent. After washing the tissue, the epithelial cell layer is scraped from the submucosa with a glass slide and minced. This is followed by incubation in trypsin or, preferably in a liquid containing at least one of EDTA and EGTA and separation of undigested tissue fragments and single cells from crypts using at least one of, for example, filtration and centrifugation. Other proteolytic enzymes, for example, at least one of collagenase and dispase I, may be used in place of trypsin. A similar method is used to isolate fragments of the pancreas and stomach.

The method for isolating a stem cell from an epithelial tissue also includes a method known in the art. The stem cell expresses on the surface thereof at least one of Lgr5 and Lgr6 (Lgr5 and Lgr6 belong to the large G protein-coupled receptor (GPCR) superfamily). The method for isolating a stem cell may be a method in which a cell suspension is prepared from an epithelial tissue, the cell suspension is brought into contact with a chemical substance which binds to at least one of Lgr5 and Lgr6, the chemical substance which binds to at least one of Lgr5 and Lgr6 is separated, and the stem cell is isolated from the binding compound.

The chemical substance that binds to at least one of Lgr5 and Lgr6 may be, for example, an antibody, more specifically, for example, a monoclonal antibody that specifically recognizes and binds to at least one of Lgr5 and Lgr6 (for example, a monoclonal antibody including a mouse and rat monoclonal antibody). Using such an antibody, it is possible to isolate a stem cell expressing at least one of Lgr5 and Lgr6, for example utilizing magnetic beads or through a fluorescence-activated cell sorter.

The epithelial stem cells, the epithelial cancer cells, or a tissue containing at least one thereof, the cells being isolated by the above-described method, is seeded on the cellular matrix obtained in the foregoing preparation step, which is then allowed to stand. The seeded cells are capable of adhering to the ECM by interaction with its surface structure, for example interaction with integrins.

### [Organoid formation step]

Subsequently, before the cells dry following the cell seeding, the cell culture medium described above is added and the cells are cultured. The culture temperature is preferably 30°C or higher and 40°C or lower, and more preferably about 37°C. The culture time can be appropriately adjusted depending on the cells to be used. An organoid can be formed about 1 to 2 weeks after the start of culture. Further, with respect to cells which could only be maintained in a cell culture for only 2 to 3 months in the past, according to the culture method of the present embodiment, the cells can be maintained in a cell culture even for a long period of time of 3 months or more (preferably about 10 months). In the case where epithelial stem cells are cultured using the culture method of the present embodiment, the differentiation potential can be maintained and the incidence of the tumor can be kept to an extremely low state.

### <<Method for culturing epithelial stem cell or tissue containing epithelial stem cell>>

In one embodiment, the present invention provides a method for culturing an epithelial stem cell or a tissue containing the epithelial stem cell, including: an extracellular matrix preparation step of preparing an extracellular matrix; an adhesion step of adhering the epithelial stem cell or the tissue containing the epithelial stem cell onto the extracellular matrix; and an organoid formation step of performing addition of a cell culture medium which is the serum-free medium as described above after the adhesion step, and culturing the epithelial stem cell or the tissue containing the epithelial stem cell to form an organoid.

According to the culture method of the present embodiment, it is possible to achieve long-term culture of an epithelial stem cell or a tissue containing an epithelial stem cell. Further, it is possible to achieve the formation of an organoid with high efficiency from an epithelial stem cell or a tissue containing an epithelial stem cell.

The method of the present embodiment will be described in detail below.

### [Extracellular matrix preparation step]

The ECM may be the same as that exemplified in the section [Extracellular matrix preparation step] of the foregoing section <<Method for culturing epithelial stem cell, epithelial cancer cell, or tissue containing these cells>>.

The preparation method of ECM may be the same as that exemplified in the section [Extracellular matrix preparation step] of the foregoing section <<Method for culturing epithelial stem cell, epithelial cancer cell, or tissue containing these cells>>.

The ECM-producing cell may be the same as that exemplified in the section [Extracellular matrix preparation step] of the foregoing section <<Method for culturing epithelial stem cell, epithelial cancer cell, or tissue containing these cells>>. Commercially available ECMs include those exemplified in the section [Extracellular matrix preparation step] of the foregoing section <<Method for culturing epithelial stem cell, epithelial cancer cell, or tissue containing these cells>>. Also, a mixture of natural ECM and synthetic ECM may be used.

The use of an ECM for culturing stem cells can enhance long-term survival of the stem cells and the continued presence of undifferentiated stem cells. In the absence of an ECM, stem cell cultures cannot be cultured over a long period of time and no continued presence of undifferentiated stem cells is observed. In addition, the presence of an ECM allows the culturing of organoids which cannot be cultured in the absence of an ECM.

The ECM is usually sunk on the bottom of the dish in which cells are suspended. For example, in the case where the ECM solidifies at 37°C, the cell culture medium described above may be added and diffused into the ECM which is then used. The cells in the medium are capable of sticking to the ECM by interaction with its surface structure, for example interaction with integrins.

The ECM may be coated on a culture vessel or the like which is then used. In the case of using fibronectin as the ECM, the culture vessel is coated with fibronectin in an amount of preferably 1 µg/cm² or more and 250 µg/cm² or less, more preferably 1 µg/cm² or more and 150 µg/cm² or less, and still more preferably 8 µg/cm² or more and 125 µg/cm² or less.

### [Adhesion step]

Subsequently, an epithelial stem cell or a tissue containing an epithelial stem cell is prepared. The epithelial stem cells used in the culture method of the present embodiment may be the same as those described in the foregoing section «Cell culture medium».

The method for isolating a stem cell from an epithelial tissue may be the same as that exemplified in the section [Adhesion step] of the foregoing section <<Method for culturing epithelial stem cell, epithelial cancer cell, or tissue containing these cells>>.

The epithelial stem cells or a tissue containing epithelial stem cells, the cells being isolated by the above-described method, is seeded on the cellular matrix obtained in the foregoing preparation step, which is then allowed to stand. The seeded cells are capable of adhering to the ECM by interaction with its surface structure, for example interaction with integrins.

### [Organoid formation step]

Subsequently, before the cells dry following the cell seeding, a cell culture medium which is the serum-free medium as described above is added and the cells are cultured. The culture temperature is preferably 30°C or higher and 40°C or lower, and more preferably about 37°C. The culture time can be appropriately adjusted depending on the cells to be used. An organoid can be formed about 1 to 2 weeks after the start of culture. Further, with respect to cells which could only be maintained in a cell culture for only 2 to 3 months in the past, according to the culture method of the present embodiment, the cells can be maintained in a cell culture even for a long period of time of 3 months or more (preferably about 10 months). In the case where epithelial stem cells are cultured using the culture method of the present embodiment, the differentiation potential can be maintained and the incidence of the tumor can be kept to an extremely low state.

### <<Organoid>>

The description describes an organoid obtainable by the foregoing culture method.

The organoid can be applied to regenerative medicine, fundamental medicine research on epithelial cells, drug response screening, drug discovery research using disease-derived epithelial organoids, and the like.

### <Uses>

The description describes the use of the above-described organoid for drug response screening, toxicity assay, or regenerative medicine.

In the case where the above-mentioned organoid is used in the drug response screening, the organoid is cultured in a multiwell plate such as a 96-well plate or a 384-well plate. A library of molecules is used to identify a molecule that affects the organoid. Examples of the library include an antibody fragment library, a peptide phage display library, a peptide library (for example, LOPAP^{™}, manufactured by Sigma-Aldrich, Inc.), a lipid library (manufactured by BioMol Inc.), a synthetic compound library (for example, LOPAP^{™}, manufactured by Sigma-Aldrich, Inc.), and a natural compound library (Specs, manufactured by TimTec Corporation). Further, a gene library may be used. Examples of the gene library include a cDNA library, an antisense library, a siRNA library, and other non-coding RNA libraries. A specific method of the drug response screenings may be, for example, a method of exposing the cells to multiple concentrations of a test agent over a certain period of time and evaluating the culture at the end of the exposure period. In addition, the organoid can also be used to identify a drug that specifically targets epithelial cancer cells and does not target an organoid consisting of normal cells.

Further, the organoid can replace the use of cell lines such as Caco-2 cells in toxicity assays of novel candidate drugs or of known or novel dietary supplements.

Further, the organoid can be used for culturing of a pathogen such as a norovirus which presently lacks a suitable tissue culture or animal model.

Further, the organoid is useful in regenerative medicine, for example in post-radiation and/or post-surgery repair of the intestinal epithelium, in the repair of the intestinal epithelium in patients suffering from inflammatory bowel disease such as Crohn's disease and ulcerative colitis, and in the repair of the intestinal epithelium in patients suffering from short bowel syndrome. Further, the organoid is useful in the repair of the intestinal epithelium in patients with hereditary diseases of the small intestine/colon. Further, the organoid is useful in regenerative medicine, for example as an implant or part thereof after pancreatectomy and for the treatment of diabetes such as type I diabetes and type II diabetes.

Further, in one embodiment, the present invention provides the use of the above-described organoid for transplantation.

The organoid obtained by the foregoing section <<Method for culturing epithelial stem cell or tissue containing epithelial stem cell>> of the present embodiment can be suitably used for transplantation since the organoid is composed of normal epithelial stem cells, maintains a differentiation potential, and has an extremely low tumor incidence.

### EXAMPLES

Hereinafter, the present invention will be described with reference to Examples, but the present invention is not limited to the following Examples.

### [Example 1]

### (1) Preparation of Wnt3a-afamin complex

Based on the fact that bovine fetal serum contains bovine afamin, the Wnt3a-afamin complex was prepared by culturing cells transfected with only Wnt3a in a medium containing serum, utilizing the fact that secreted Wnt3a automatically forms a stable complex with afamin. That is, cells expressing Wnt3a having a tag sequence at the N terminus (W-Wnt3a/HEK) are cultured in a medium containing 10% fetal bovine serum in a dish or multilayered flask for 5 to 7 days, and the culture supernatant was recovered. Subsequently, the recovered culture supernatant was centrifuged and passed through a filter (0.22 µm). 220 mL of the collected culture supernatant was used. Subsequently, 3 mL of P20.1 antibody-sepharose was added to 200 mL of the recovered culture supernatant, followed by rotational mixing at 4°C for 3 hours, and the medium was passed through an empty column to collect the sepharose. Note that the P20.1 antibody is an antibody that specifically recognizes the N-terminal tag sequence of Wnt3a. Subsequently, the sepharose collected in the column was washed with 3 mL of Tris bufferd saline (20 mM Tris-HCl, 150 mM NaCl, pH 7.5). This washing step was repeated 5 times. Subsequently, elution was carried out using 3 mL of a peptide solution (0.2 mg/mL PAR4-C8 peptide/TBS) each time, and the eluate was recovered. The elution step was repeated 10 times to obtain a Wnt3a-afamin complex. The activity of Wnt3a was confirmed by TCF reporter assay using the cell supernatant of W-Wnt3a/HEK, and it was confirmed that the activity was 10 times or more higher than that of commercially available Wnt3a (manufactured by R&D Systems Inc.).

### (2) Preparation of cell culture medium

To a commercially available Advanced DMEM/F-12 medium (manufactured by Thermo Ficher Scientific Inc.), human recombinant R-spondin 1 (manufactured by R&D Systems Inc.) was added to a final concentration of 1 µg/mL, Noggin (manufactured by PeproTech Inc.) was added to a final concentration of 100 ng/mL, A83-01 (manufactured by Tocris Bioscience Inc.) was added to a final concentration of 500 nM, and EGF (manufactured by Thermo Ficher Scientific Inc.) was added to a final concentration of 50 ng/mL, and SB202190 (manufactured by Sigma-Aldrich, Inc.) was added to a final concentration of 10 µM (ENRAS medium). In addition, a medium in which the Wnt3a-afamin complex (hereinafter, referred to as "rWnt") prepared in the section (1) was added to a final concentration of 300 ng/mL or 1,000 ng/mL, or a medium in which commercially available Wnt3a (manufactured by R&D Systems Inc.) (hereinafter, referred to as "R&D") was added to a final concentration of 300 ng/mL was prepared. In addition, as controls, a medium not containing Wnt3a (hereinafter, referred to as "Wnt (-)"), and a medium to which the culture supernatant derived from W-Wnt3a/HEK cultured in a serum-containing medium was added so that the final concentration of Wnt3a was 300 ng/mL (hereinafter, referred to as "Wnt CM") were prepared.

### (3) Culture of intestinal stem cells

Based on the Ethics Research Program approved at Keio University School of Medicine Ethics Committee, from colorectal tumor patients with explanation and consent, a portion at least 5 cm or more away from the colorectal tumor was collected as a normal mucous membrane and a colorectal cancer tissue was collected from the colorectal tumor. The harvested tissues were treated with EDTA or Liberase TH to extract epithelial cells which were then embedded in Matrigel (registered trademark).

The Matrigel (registered trademark) containing epithelial cells (hereinafter, referred to as "intestinal stem cells") was seeded onto a 24-, 48-, or 96-well plate and cultured with the medium. Specifically, it is as follows.

The cultured intestinal stem cells were seeded onto a 96-well plate together with 10 µL of Matrigel (registered trademark, manufactured by BD Biosciences, Inc.). 100 µL/well of each of the 6 media prepared in the section (2) (rWnt 300ng/mL, rWnt 1000ng/mL, R&D 300ng/mL, Wnt CM, and Wnt(-)) was added, followed by culturing at 37°C. Medium exchange was carried out every 2 days from the culture. FIG. 1(A) is an image showing a state after 6 days from the start of culture of the primary culture (Passage 0), FIG. 2(A) is an image showing a state after 6 days from the start of culture at the first passage (Passage 1), and FIG. 3(A) is an image showing a state after 5 days from the start of culture at the second passage (Passage 2). FIG. 1(B), FIG. 2(B), and FIG. 3(B) are graphs obtained by quantifying the area occupied by the organoids of intestinal stem cells in wells in each medium.

From FIGS. 1 to 3, it was confirmed that intestinal stem cells can be subcultured for a long period of time in a medium containing the Wnt3a-afamin complex. In addition, although data are not shown, in the medium containing the Wnt3a-afamin complex, the intestinal stem cells could be subcultured with 18 passages or more for a period of 4 months.

### [Example 2]

### (1) Preparation of cell culture medium

To a commercially available Advanced DMEM/F-12 medium (manufactured by Thermo Ficher Scientific Inc.), EGF (manufactured by Thermo Ficher Scientific Inc.) was added to a final concentration of 50 ng/mL, Noggin (manufactured by PeproTech Inc.) was added to a final concentration of 100 ng/mL, and A83-01 (manufactured by Tocris Bioscience Inc.) was added to a final concentration of 500 nM (hereinafter, referred to as "ENA medium").

In addition, to a commercially available Advanced DMEM/F-12 medium (manufactured by Thermo Ficher Scientific Inc.), EGF (manufactured by Thermo Ficher Scientific Inc.) was added to a final concentration of 50 ng/mL, Noggin (manufactured by PeproTech Inc.) was added to a final concentration of 100 ng/mL, A83-01 (manufactured by Tocris Bioscience Inc.) was added to a final concentration of 500 nM, and SB202190 (manufactured by Sigma-Aldrich, Inc.) was added to a final concentration of 10 µM (hereinafter, referred to as "ENAS medium").

A medium used in the conventional method was prepared by adding commercially available Wnt3a (manufactured by R&D Systems Inc.) was added to the ENAS medium to a final concentration of 300 ng/mL.

Further, the cell culture medium of the present invention was prepared by adding the Wnt3a-afamin complex prepared in the section (1) of Example 1 to each of ENA medium and ENAS medium to a final concentration of 300 ng/mL. As controls, an ENA medium and an ENAS medium each not containing the Wnt3a-afamin complex were also prepared.

### (2) Culture of epithelial cancer cells derived from colorectal cancer

Using the same method as in the section (3) of Example 1, the large intestine containing epithelial cancer cells derived from a human tumor tissue was divided into epithelial cancer cells and remaining tissues using Liberase TH. The remaining tissues were further decomposed with trypsin. Subsequently, epithelial cancer cells, or epithelial cells and the remaining tissues decomposed apart were seeded onto 96-well plates together with 10 µL of Matrigel (registered trademark, manufactured by BD Biosciences, Inc.). 100 µL of the ENAS medium containing commercially available Wnt3a (a medium used in the conventional method) prepared in the section (2) was added to wells seeded with epithelial cancer cells. In addition, 100 µL of each of the ENA medium and the ENAS medium each supplemented with the Wnt3a-afamin complex, and the ENA medium and the ENAS medium each not containing Wnt3a prepared in the section (2) was added to wells seeded with epithelial cells and the remaining tissues decomposed apart.

Subsequently, epithelial cancer cells to which the ENAS medium containing commercially available Wnt3a (a medium used in the conventional method) was added, and epithelial cancer cells to which the ENA medium and the ENAS medium prepared in the section (2) each supplemented with the Wnt3a-afamin complex were added were cultured.

As a result, in the case of epithelial cancer cells to which the ENAS medium containing commercially available Wnt3a (a medium used in the conventional method) was added, the establishment efficiency of the colorectal cancer organoid was 67.5%, whereas in the case of the ENA medium and the ENAS medium prepared in the section (2) each supplemented with the Wnt3a-afamin complex, the establishment efficiency of the colorectal cancer organoid was 100.0%.

From the foregoing, it was demonstrated that the cell culture medium of the present invention is capable of forming an organoid with high efficiency in colorectal cancer-derived epithelial cancer cells.

### [Example 3]

### (1) Preparation of cell culture medium

First, using the same method as in the section (1) of Example 2, an ENRAS+Wnt3a-AFM medium in which the Wnt3a-afamin complex prepared in the section (1) of Example 1 was added to a final concentration of 300 ng/mL and human recombinant R-spondin 1 (manufactured by R&D Systems Inc.) was added to a final concentration of 1 µg/mL was prepared.

### (2) Scrape of normal epithelial stem cells from NOG mouse

First, a sonde with a balloon attached to the tip was constructed (reference: Fukuda et al., Genes and Development, 28, 1752-1757, 2014). Next, to the NOD/Shi-scid-IL2Rγ^{null} mouse (NOG mouse), which is a hyperimmune-deficient mouse, under inhalation anesthesia, the sonde was inserted 1 to 2 cm from the mouse anus, and the balloon was inflated so that the mouth of the rectum was clamped. Next, warmed EDTA was administered into the intestinal lumen formed between the anus and the rectum (see upper left panel of FIG. 4), whereby the lumen was closed over 2 to 3 minutes. Subsequently, the balloon was removed, and one side of the rectal epithelium was rubbed with a brush, whereby the epithelium which became easier to be scraped by EDTA was exfoliated including the bottom of the crypt (see upper right panel and lower left and right panels of FIG. 4).

### (3) Culture of epithelial stem cells

Subsequently, using the ENRAS+Wnt3a-AFM medium prepared in the section (1), human epithelial stem cells were cultured to construct a human colorectal organoid which was then further cultured for 3 to 4 days to grow the human colorectal organoid.

### (4) Transplantation of human colorectal organoid into NOG mouse

About 10⁶ to 10⁸ cells were then suspended in 50 to 200 µL of PBS containing 5 to 20% Matrigel. Subsequently, a suspension containing about 10⁶ to 10⁸ cells per mouse was injected from the anus of the mucosally exfoliated NOG mouse, so that the anus of the animal was temporarily closed (see left panel of FIG. 5). In addition, the human colorectal organoid was labeled with GFP in advance. Two weeks after transplantation, and 16 weeks after transplantation, the transplantation site of the NOG mouse was observed in the bright field and fluorescence mode using an endoscope. The results are shown in FIG. 5.

From FIG. 5, the fluorescence of GFP was observed at the transplantation site. As a result, it was confirmed that GFP labeling of the human colorectal organoid in advance enables the observation over time after engraftment of the organoid. Also, although not shown, long-term engraftment of the organoid for at least half a year or longer was recognized.

Using the NOG mouse at 28 days post-transplantation, the transplantation site was cut out and pathological tissues were observed. The results are shown in FIG. 6. In FIG. 6, the upper panel is an image showing the results of hematoxylin-eosin (HE) staining of a tissue section. The lower left panel is an image showing the results of immunostaining and nuclear staining of the tissue section, with an anti-human cytokeratin antibody and Hoechst (registered trademark), respectively. The lower right panel is an image showing the results of the detection of Lgr5 by in situ hybridization analysis of the tissue section, and nuclear staining of that tissue section with Hoechst (registered trademark).

From FIG. 6, by means of HE staining, the crypt formed by the engraftment of the human colorectal organoid was identified as a larger crypt, as compared to the crypt of the mouse. In addition, immunostaining using an anti-human cytokeratin antibody revealed that the crypt formed by the engraftment of the human colorectal organoid is due to human cells.

Further, based on the detection results of Lgr5 by in situ hybridization analysis, it was demonstrated that Lgr5, which is an intestinal stem cell marker, is expressed in the bottom of the crypt formed by the engraftment of the transplanted human colorectal organoid and is supported by the mouse stroma, so that the human intestinal epithelial stem cells (organoids) function, thus resulting in long-term engraftment thereof.

From the above results, it was suggested that the animal model transplanted with the organoid of the present invention can be applied to regenerative medicine, fundamental medicine research on epithelial cells, drug response screening, drug discovery research using disease-derived epithelial organoids, and the like.

### Industrial Applicability

According to the cell culture medium of the present invention, it is possible to achieve long-term culture of an epithelial stem cell, an epithelial cancer cell, or a tissue containing at least one thereof, the cell being derived from a mammal including a human, or a tissue that could not be cultured in the past. Further, it is possible to achieve the formation of an organoid with high efficiency from at least one of the cell and the tissue. In addition, epithelial stem cells cultured using the cell culture medium of the present invention can maintain a differentiation potential for a long period of time, and exhibit a very low tumor incidence. Further, the organoids obtained by the culture method of the present invention can be applied to regenerative medicine, fundamental medicine research on epithelial cells, drug response screening, drug discovery research using disease-derived epithelial organoids, and the like.

## Claims

1. A serum-free cell culture medium comprising:
a Wnt agonist including a complex of a Wnt protein and an afamin, and an R-spondin; and
at least one selected from the group consisting of a mitogenic growth factor, a bone morphogenetic protein (BMP) inhibitor, a transforming growth factor-β (TGF-β) inhibitor, and a p38 inhibitor.

2. The serum-free cell culture medium according to Claim 1, wherein the concentration of Wnt protein is 300 ng/mL or more.

3. The serum-free cell culture medium according to Claim 1 or 2, wherein the activity of the purified complex of a Wnt protein and an afamin is 10 times or more higher than that of Wnt protein according to the T-cell Factor (TCF) reporter assay.

4. The serum-free cell culture medium according to any one of Claims 1 to 3, wherein the Wnt protein is at least one selected from the group consisting of Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16.

5. The serum-free cell culture medium according to any one of Claims 1 to 4, wherein the R-spondin is at least one selected from the group consisting of R-spondin 1, R-spondin 2, R-spondin 3, and R-spondin 4.

6. The serum-free cell culture medium according to any one of Claims 1 to 5, wherein the mitogenic growth factor is an epidermal growth factor (EGF).

7. The serum-free cell culture medium according to any one of Claims 1 to 6, wherein the BMP inhibitor is Noggin.

8. The serum-free cell culture medium according to any one of Claims 1 to 7, wherein the TGF-β inhibitor is A83-01.

9. A method for culturing an epithelial stem cell, an epithelial cancer cell, or a tissue containing at least one of the cells, comprising:
an extracellular matrix preparation step of preparing an extracellular matrix;
an adhesion step of adhering the epithelial stem cell, the epithelial cancer cell, or the tissue containing at least one of the cells onto the extracellular matrix; and
an organoid formation step of performing addition of the serum-free cell culture medium according to any one of claims 1 to 8 after the adhesion step, and culturing the epithelial stem cell, the epithelial cancer cell, or the tissue containing at least one of the cells to form an organoid.

## Patentansprüche

1. Serumfreies Zellkulturmedium, umfassend:
einen Wnt-Agonisten, der einen Komplex aus einem Wnt-Protein und einem Afamin und ein R-spondin beinhaltet; und
mindestens eines, das aus der Gruppe ausgewählt ist, die aus einem mitogenen Wachstumsfaktor, einem Inhibitor des knochenmorphogenetischen Proteins (BMP), einem Inhibitor des Transforming Growth Factor β (TGF-β) und einem p38-Inhibitor besteht.

2. Serumfreies Zellkulturmedium nach Anspruch 1, wobei die Konzentration des Wnt-Proteins 300 ng/ml oder mehr beträgt.

3. Serumfreies Zellkulturmedium nach Anspruch 1 oder 2, wobei die Aktivität des gereinigten Komplexes aus einem Wnt-Protein und einem Afamin gemäß der T-Cell-Faktor(TCF)-Reporter-Auswertung 10 mal oder mehr größer ist als die des Wnt-Proteins.

4. Serumfreies Zellkulturmedium nach einem der Ansprüche 1 bis 3, wobei das Wnt-Protein mindestens eines ist, das aus der Gruppe ausgewählt ist, die aus Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11 und Wnt 16 besteht.

5. Serumfreies Zellkulturmedium nach einem der Ansprüche 1 bis 4, wobei das R-spondin mindestens eines ist, das aus der Gruppe ausgewählt ist, die aus R-spondin 1, R-spondin 2, R-spondin 3 und R-spondin 4 besteht.

6. Serumfreies Zellkulturmedium nach einem der Ansprüche 1 bis 5, wobei der mitogene Wachstumsfaktor ein epidermaler Wachstumsfaktor (EGF) ist.

7. Serumfreies Zellkulturmedium nach einem der Ansprüche 1 bis 6, wobei der BMP-Inhibitor Noggin ist.

8. Serumfreies Zellkulturmedium nach einem der Ansprüche 1 bis 7, wobei der TGF-β-Inhibitor A83-01 ist.

9. Verfahren zum Kultivieren einer epithelialen Stammzelle, einer epithelialen Krebszelle oder eines Gewebes, das mindestens eine der Zellen enthält, umfassend:
einen extrazellulären Matrixvorbereitungsschritt eines Vorbereitens einer extrazellulären Matrix;
einen Adhäsionsschritt eines Anheftens der epithelialen Stammzelle, der epithelialen Krebszelle oder des Gewebes, das mindestens eine der Zellen enthält, an die extrazelluläre Matrix; und
einen Organoidbildungsschritt eines Durchführens einer Zugabe des serumfreien Zellkulturmediums nach einem der Ansprüche 1 bis 8 nach dem Adhäsionsschritt und Kultivierens der epithelialen Stammzelle, der epithelialen Krebszelle oder das Gewebe, das mindestens eine der Zellen enthält, um ein Organoid zu bilden.

## Revendications

1. Milieu de culture cellulaire sans sérum comprenant :
un agoniste de Wnt comprenant un complexe d'une protéine Wnt et d'une afamine, et une R-spondine ; et
au moins un choisi parmi le groupe constitué d'un facteur de croissance mitogène, d'un inhibiteur de la protéine morphogénétique osseuse (BMP), d'un inhibiteur du facteur de croissance transformant-β (TGF-β) et d'un inhibiteur de p38.

2. Milieu de culture cellulaire sans sérum selon la revendication 1, dans lequel la concentration de la protéine Wnt est de 300 ng/mL ou plus.

3. Milieu de culture cellulaire sans sérum selon la revendication 1 ou 2, dans lequel l'activité du complexe purifié d'une protéine Wnt et d'une afamine est au moins 10 fois plus élevée que celle de la protéine Wnt selon le test rapporteur du facteur des lymphocytes T (TCF).

4. Milieu de culture cellulaire sans sérum selon l'une quelconque des revendications 1 à 3, dans lequel la protéine Wnt est au moins une choisie parmi le groupe constitué de Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, et Wnt16.

5. Milieu de culture cellulaire sans sérum selon l'une quelconque des revendications 1 à 4, dans lequel la R-spondine est au moins une choisie parmi le groupe constitué de R-spondine 1, R-spondine 2, R-spondine 3, et R-spondine 4.

6. Milieu de culture cellulaire sans sérum selon l'une quelconque des revendications 1 à 5, dans lequel le facteur de croissance mitogène est un facteur de croissance épidermique (EGF).

7. Milieu de culture cellulaire sans sérum selon l'une quelconque des revendications 1 à 6, dans lequel l'inhibiteur de BMP est la noggine.

8. Milieu de culture cellulaire sans sérum selon l'une quelconque des revendications 1 à 7, dans lequel l'inhibiteur du TGF-β est A83-01.

9. Procédé permettant la culture d'une cellule souche épithéliale, d'une cellule cancéreuse épithéliale ou d'un tissu contenant au moins une des cellules, comprenant :
une étape de préparation de matrice extracellulaire de préparation d'une matrice extracellulaire ;
une étape d'adhérence de la cellule souche épithéliale, de la cellule cancéreuse épithéliale ou du tissu contenant au moins une des cellules sur la matrice extracellulaire ; et
une étape de formation organoïde d'ajout du milieu de culture cellulaire sans sérum selon l'une quelconque des revendications 1 à 8 après l'étape d'adhérence et de culture de la cellule souche épithéliale, de la cellule cancéreuse épithéliale, ou du tissu contenant au moins une des cellules pour former un organoïde.
